# EUROPEAN PATENT SPECIFICATION

(11) **EP 0 557 303 B1**
(45) Date of publication and mention of the grant of the patent: **14.09.1994**
(21) Application number: 91918033.1
(22) Date of filing: 30.10.1991
(51) Int. Cl.: C07D 333/38, A61K 31/38

(54) **N- (2-THENOYLMERCAPTO-3-METHYL)-BUTANOYL]-HOMOCYSTEINE THIOLACTONE, A PROCESS FOR THE PREPARATION THEREOF AND PHARMACEUTICAL COMPOSITIONS CONTAINING IT**
N-[(2-THENOYLMERKAPTO-3-METHYL)-BUTANOYL]-HOMOCYSTEINTHIOLACTON, VERFAHREN ZU SEINER HERSTELLUNG UND DIESE ENTHALTENDE PHARMACEUTISCHE ZUSAMMENSETZUNGEN
N- (2-THENOYLMERCAPTO-3-METHYLE)-BUTANOYLE]-HOMOCYSTEINE THIOLACTONE, UN PROCEDE SERVANT A SA PREPARATION ET COMPOSITIONS PHARMACEUTIQUES LE CONTENANT

(30) Priority: 12.11.1990 IT 2201590
(43) Date of publication of application: 01.09.1993
(73) Proprietor: MEDEA RESEARCH S.r.l., I-20122 Milano (IT)
(72) Inventor: QUADRO, Giuseppe, I-20129 Milano (IT)
(74) Representative: Bianchetti, Giuseppe
(86) International application number: EP9102052
(87) International publication number: WO9208713

(56) References cited:
- EP-A- 0 120 534

## Description

The present invention relates to a novel thiobutyrolactone derivative, namely N-[(2-thenoylmercapto-3-methyl)-butanoyl]-homocysteine thiolactone, the enantiomers and diastereoisomers thereof, as well as a process for the preparation thereof and pharmaceutical compositions containing it.

Cysteine and homocysteine derivatives have bronchosecretolytic activity. γ-Butyrolactones are homocysteine precursors, in which the thiol group is made available in vivo, by means of cleavage of the thiolactone ring.

2-(2-Thenoylthio)-N-(2',3',4',5'-tetrahydro-2'-keto-thiophen-3'-yl)propionyl amide is known from the following patents: It. Pat. N. 1,212,723, EP-Pat. N. 0,120,534, USA-Pat. N. 4,481,212, wherein the bronchosecretolytic activity of the compound is disclosed. Subsequently, the same compound, also named MR-889, has also been found to have important elastase inhibiting properties, as disclosed in Biochemical Pharmacology, Vol. 39, No. 5, pp. 919-924, 1990 and in Biochemical and Biophysical research communications Vol. 165, No. 15, pp. 568-573, 1989.

In view of said enzyme inhibiting properties, a novel homocysteine derivative has been developed.

The compound of the invention, which will hereinafter be named MR-2047, is structurally related to MR-889 but, besides having some of the biochemical and pharmacological properties of MR-889, it surprisingly also has an unforeseeable collagenase inhibiting activity which is totally absent in the case of MR-889.

Said properties make MR-2047 useful in the treatment of collagen degenerative processes, arthrosis, pulmonary fibrosis, arteriosclerosis, atherosclerosis and also in some metastatic conditions.

The biological activities of MR-889 and MR-2047 have been compared.

### Elastase inhibiting activity.

The inhibiting activity on swine pancreatic elastase (Boehringer) was tested according to the method described by J. Aaron (Biochem. J. 114: 157-159; 1969), using the synthetic peptide benzyloxycarbonyl-L-alanine p-nitrophenyl ester (Sigma) as the substrate. Under the used experimental conditions, a remarkable elastase inhibiting activity was evidenced for both compounds, with an IC₅₀ value of about 6 µM.

### Collagenase inhibiting activity.

The proteolytic activity of collagenase from Clostridium Histolyticum (Sigma, type II) was dosed according to the method by Appel (Method in enzymatic analysis, H.U. Bergmayer Vol. 2, pp. 1058-1063; 1978) using the collagen synthetic peptide N-carbobenzoxy-pro-gly-gly-L-pro-L-alanine as the substrate. Under the used experimental conditions, MR-889 showed almost no inhibiting activity, whereas MR-2047 proved to have a marked activity with an IC₅₀ of about 2 µM.

### Antiarteriosclerotic activity.

The antiarteriosclerotic activity was tested according to the procedure described by C.T. Cahn, Circul. Research, 43, 115, 1975. The procedure consists in verifying any protecting actions of a drug on arteriosclerotic lesions induced by the diet in the rabbit. At the end of a 12 week treatment, the group of animals administered with MR-2047 together with the diet showed a significant reduction in cholesterol and triglyceride levels, as well as in collagen and elastin levels in the connective tissue of the aorta intima.

The compound of the invention can be prepared according to conventional methods, by reacting 2-bromo-3-methylbutyric acid with NaHS, to obtain 2-mercapto-3-methylbutyric acid (A), which is subsequently reacted with 2-thenoic acid, to give 2-thenoylthio-3-methylbutyric acid (B).

Compound (B) is preferably obtained by reacting compound (A) with a 2-thenoic acid reactive derivative, such as the acid chloride or the anhydride. The reaction of (A) with 2-thenoyl chloride in basic medium, such as sodium hydroxide, is particularly preferred. The reaction temperature is from 0 to 25°C, preferably from 5 to 20°C. The compound of the invention is obtained by reacting an homocysteine thiolactone salt with 2-thenoylthio-3-methylbutyric acid an anhydrous solvent.

Preferably, condensation of homocysteine thiolactone with 2-thenoylthio-3-methylbutyric acid is carried out using a reactive derivative of the latter, such as an acid halide or an anhydride. Particularly preferred is the acid chloride, which is prepared conventionally with thionyl chloride.

Preferred solvents are acetic acid C₁-C₃ esters, such as ethyl acetate. The reaction temperature is from -5 to 25°C, preferably from 5 to 20°C.

The process for the preparation of MR-2047 is summarized in the following reaction scheme : 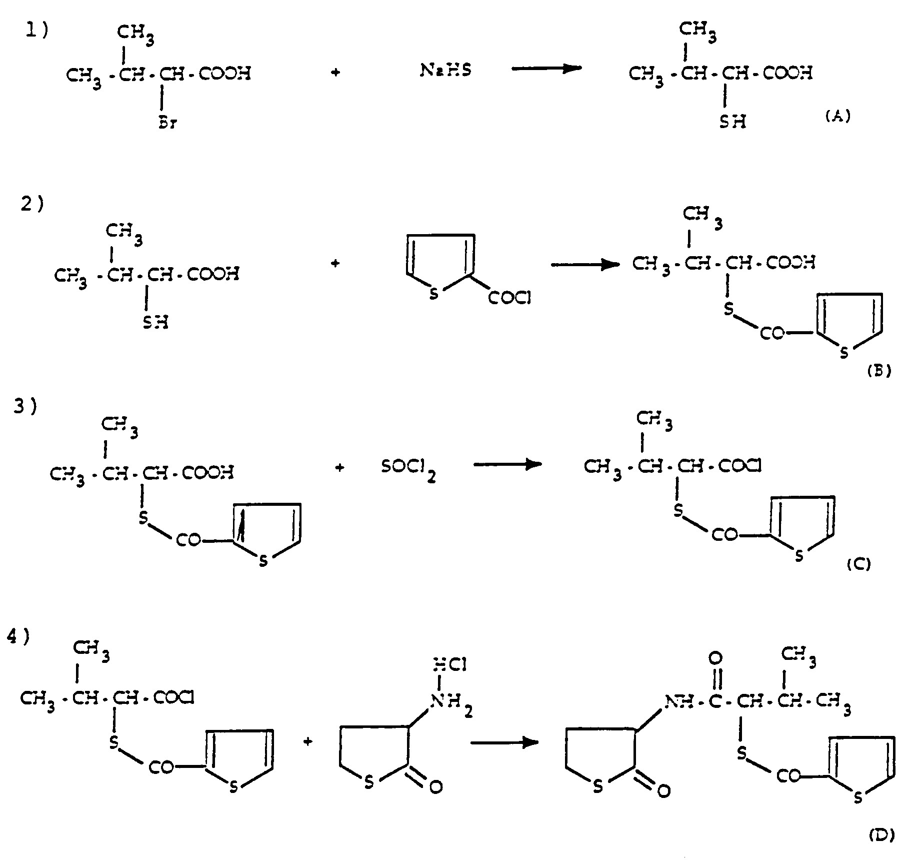
The following Example further illustrates the invention.

### EXAMPLE

### 1) Preparation of (A).

15 g (0.083 mole) of 2-bromoisovaleric acid are added in portions to a solution of 17 g (0.23 mole) of 90% NaHS H₂O and 40 ml of water. The solution is heated to 100°C for 3 hours, then it is cooled to 10°C and acidified to pH 2 with conc. HCl. The reaction mixture is extracted with 2 x 40 ml of ethyl ether. The extracts are dried over sodium sulfate and evaporated, to obtain (A) in form of a colourless liquid which slowly solidifies at room temperature.
- Yield: 9.44 g (85%)
- TLC (eluent: toluene/dioxane/acetic acid = 45/10/2; development in I₂; unitary Rf ≃ 0.5)
- N.M.R. (CDCl₃): in agreement
- M.p. lit.: 35° C

### 2) Preparation of (B).

9.15 g (0.068 mole) of (A) are dissolved in 60 ml of water containing 5.74 g (0.136 mole) of NaOH. The solution is cooled to 5°C, then 7.3 ml (≡ 10 g, 0.068 mole) of thiophenecarbonyl chloride are dropped therein, keeping said temperature. At the end of the addition, the reaction mixture is left to warm to room temperature and to react for 4 hours, then it is cooled to 5°C, acidified to pH 2 with conc. HCl and extracted with 3 x 20 ml of methylene chloride. The extracts are dried and the solvent is evaporated off, to obtain (B) as a viscous colourless liquid.
- Yield: 15.86 g (96%)
- N.M.R. (CDCl₃): in agreement
- T.L.C. (eluent: see (A): unitary)

### 3) Preparation of (C).

5 g (0.02 mole) of (B) and 10 ml of thionyl chloride are mixed and left to react at room temperature for 16 hours. The excess thionyl chloride is evaporated off, the residue is taken up in toluene and evaporated to dryness again, to obtain (C) as a yellow liquid which is used directly in the subsequent step. Yield: nearly quantitative.

### 4) Preparation of (D).

3.07 g (0.02 mole) of D,L-homocysteine thiolactone are suspended in 40 ml of ethyl acetate, then 5.53 ml (0.04 mole) of triethylamine and a small amount of Bu₄N⁺HSO₄⁻ are added thereto. The reaction mixture is cooled to 0 - 5°C and 0.02 theoretical mole of (C), dissolved in 20 ml of ethyl acetate, are dropped therein, keeping said temperature. The mixture is left to warm to room temperature and to react for 5 hours, then it is washed with 20 ml of water, 20 ml of 5% NaHCO₃, 20 ml of 5% HCl, 20 ml of water, in this succession. After that, the mixture is dried over sodium sulfate and the solvent is evaporated off, to obtain 5.86 g of a yellow semi-solid which is left under stirring for one hour with 35 ml of a 6/1 diisopropyl ether/isopropanol mixture, to obtain a colourless crystalline solid (D) which is filtered and washed with the same mixture, to obtain 3.27 g of (D). Mother liquors still contain some (D), therefore they are evaporated and the residue is chromatographed on 50 g of SiO₂, using 9/1 toluene/ethyl acetate as the eluent. The fractions containing (D), Rf ≃ 0.65, are recovered and evaporated, to yield a yellow semi-solid which, upon treatment with 1/6 isopropanol/diisopropyl ether, gives 0.8 g of (D) as a colourless solid.
- Yield: 3.27 + 0.8 = 4.07 g (59%)
- M.p.: 104-106°C
- T.L.C. (9/1 toluene/ethyl acetate, unitary, Rf ≃ 0.65)

| Elemental analysis (for C₁₄H₁₇NO₃S₃) | | | |
|---|---|---|---|
| | C | H | N |
| % calc. | 48.98 | 4.97 | 4.08 |
| % found | 48.93 | 5.04 | 4.12 |

- N.M.R. (CDCl₃)
   NMR Analysis evidences that (D) consists of an about 1:1 diastereoisomeric mixture, as both the starting materials (bromoisovaleric acid and homocysteine thiolactone) were racemates.
   1.05 δ (2d; 6H) - 1.7-3.4 δ (4m; 5H) - 4.0 and 4.05 δ (2d; 1H) - 4.3-4.7 δ (m; 1H) - 6.7 δ (seg. all.; 1H) - 7.1 δ (d; 1H) - 7.6 and 7.8 δ (d; 2H).

## Claims (Claims for the following Contracting State(s): AT, BE, CH, LI, DE, DK, FR, GB, IT, LU, NL, SE)

1. N-[(2-Thenoylmercapto-3-methyl)-butanoyl]-homocysteine thiolactone of formula 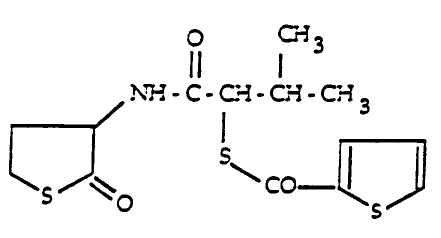 and the enantiomers and diastereoisomers thereof.

2. A process for the preparation of the compound of claim 1, characterized in that homocysteine thiolactone hydrochloride is reacted with 2-thenoylthio-3-methylbutyric acid chloride.

3. The use of the compound of claim 1 as a therapeutic agent.

4. Pharmaceutical compositions containing the compound of claim 1 as the active ingredient.

5. The use of the compound of claim 1 for the preparation of a medicament having anticollagenase activity.

## Claims (Claims for the following Contracting State(s): ES, GR)

1. A process for the preparation of N-[(2-thenoylmercapto-3-methyl)-butanoyl]-homocysteine thiolactone of formula 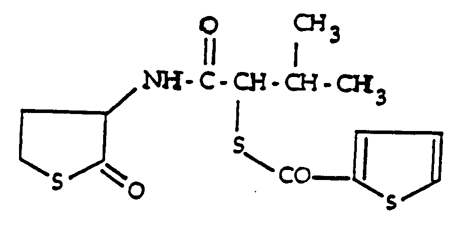 and the enantiomers and diastereoisomers thereof, characterized in that homocysteine thiolactone hydrochloride is reacted with 2-thenoylthio-3-methylbutyric acid chloride.

## Patentansprüche (Patentansprüche für folgende(n) Vertragsstaat(en): AT, BE, CH, LI, DE, DK, FR, GB, IT, LU, NL, SE)

1. N-[(2-Thenoylmercapto-3-methyl)butanoyl]homocysteinthiolacton der Formel 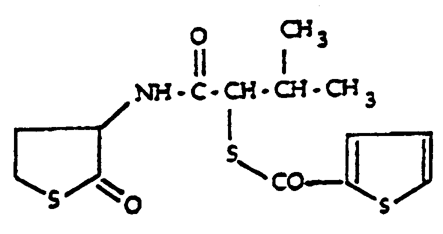 und die Enantiomeren und Diastereoisomeren davon.

2. Verfahren zur Herstellung der Verbindung nach Anspruch 1, dadurch **gekennzeichnet**, daß Homocysteinthiolacton-Hydrochlorid mit 2-Thenoylthio-3-methylbuttersäurechlorid umgesetzt wird.

3. Die Verwendung der Verbindung nach Anspruch 1 als therapeutisches Mittel.

4. Pharmazeutische Zubereitungen, dadurch **gekennzeichnet**, daß sie die Verbindung nach Anspruch 1 als aktiven Bestandteil enthalten.

5. Die Verwendung der Verbindung nach Anspruch 1 für die Herstellung eines Arzneimittels mit Anticollagenase-Aktivität.

## Patentansprüche (Patentansprüche für folgende(n) Vertragsstaat(en): ES, GR)

1. Verfahren zur Herstellung von N-[(2-Thenoylmercapto-3-methyl)butanoyl]homocysteinthiolacton der Formel 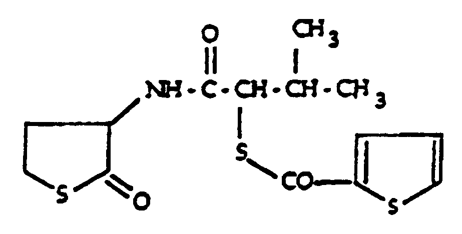 und den Enantiomeren und Diastereoisomeren davon, dadurch **gekennzeichnet**, daß Homocysteinthiolacton-Hydrochlorid mit 2-Thenoylthio-3-methylbuttersäurechlorid umgesetzt wird.

## Revendications (Revendications pour l'(les) Etat(s) contractant(s) suivant(s): AT, BE, CH, LI, DE, DK, FR, GB, IT, LU, NL, SE)

1. N-[(2-thénoylmercapto-3-méthyl)-butanoyl]-homocystéine thiolactone de formule : 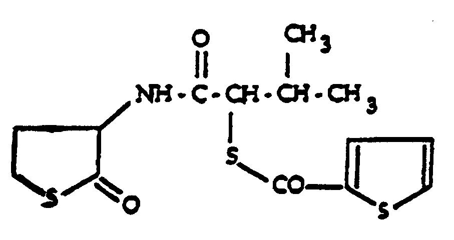 et ses énantiomères et diastéréoisomères.

2. Procédé de préparation du composé de la revendication 1, caractérisé en ce que le chlorhydrate de l'homocystéine thiolactone est mis à réagir avec le chlorure de l'acide 2-thénoylthio-3-méthylbutyrique.

3. Utilisation du composé de la revendication 1 en tant qu'agent thérapeutique.

4. Compositions pharmaceutiques contenant le composé de la revendication 1 en tant que principe actif.

5. Utilisation du composé de la revendication 1 pour la préparation d'un médicament ayant une activité anticollagènase.

## Revendications (Revendications pour l'(les) Etat(s) contractant(s) suivant(s): ES, GR)

1. Procédé de préparation de la N-[(2-thénoylmercapto-3-méthyl)-butanoyl]-homocystéine thiolactone de formule : 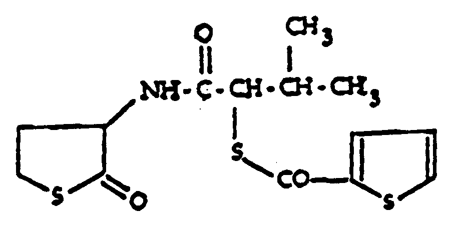 et de ses énantiomères et diastéréoisomères, caractérisé en ce que le chlorhydrate d'homocystéine thiolactone est mis à réagir avec le chlorure de l'acide 2-thénoylthio-3-méthylbutyrique.
